# EUROPEAN PATENT APPLICATION

(11) **EP 3 821 932 A1**
(43) Date of publication of application: **19.05.2021**
(21) Application number: 19220017.8
(22) Date of filing: 30.12.2019
(51) Int. Cl.: A61M 16/10, A61M 16/20, A61M 16/08

(54) **LINKING ELEMENT FOR LINKING A HOSE BARB COMPONENT**

(30) Priority: 17.11.2019 EP 19209612
(71) Applicant: Oxypoint NV, 2018 Antwerpen (BE)
(72) Inventor: HENDRICKX, Philip, 2600 Berchem (BE); VAN STEENKISTE, Cedric, 2547 Lint (BE); PINTENS, Niels, 2900 Schoten (BE); BORGONJON, Dirk, 2950 Kapellen (BE); VAN ROOST, Wouter, 1981 Hofstade (BE); HERMANS, Paul, 2660 Hoboken (BE)
(74) Representative: DenK iP bv

(57) **Abstract**

A gas valve system adapted for controlling the flow of a medical gas for a user, the gas valve system (100) comprising an input portion (120) for connecting the gas valve to an external supply, a regulating system (130) for regulating the flow of the gas and an output portion for providing the controlled flow of the medical gas towards the user, the output portion having a tubular shaped ending with a threaded portion for releasably connecting a hose barb component suitable for connecting tubing to the gas valve. In the gas valve system, the output portion furthermore has a protrusion upstream the threaded portion, and the system comprises a linking element (110) being connected to the output portion of the gas valve system upstream the protrusion and being suitable for fixedly linking the hose barb component to the output portion of the gas valve system.

## Description

### Field of the invention

The invention relates to the medical field. More particularly, the present invention relates to systems and methods for administering gas to a patient, e.g. using a nasal cannula or breathing mask.

### Background of the invention

The use of gases in medical applications is widespread. One of the gases often administered to patients for medical reasons is oxygen. In view of the fact that administering oxygen is often essential for preventing damage to tissue, avoiding life-threatening situations or saving a patient from a life-threatening situation, hospitals distribute oxygen using a pipeline network up to the bed of nearly each patient. Furthermore, oxygen therapy is also widespread for homecare patients, making use of pressurised oxygen bottles and or oxygen concentrators. In both cases, a gas valve system is used for controlling the flow, i.e. between 1 I/min and 15 I/min or higher, and the type of delivery, i.e. continuous flow or on demand flow. Gas valve systems exist both in full mechanical as well as in combined mechanical and electronic configurations.

Medical gasses typically are administered to patients using a nasal cannula or a mouth mask whereby nasal cannulas often are preferred in view of patient comfort. In order to connect the nasal cannula or the mouth mask to the gas valve, the gas valve system typically comprises a hose barb component. Such hose barb components are well known to the person skilled in the art. They typically are provided on one side with a threaded portion for connected, in a substantially gas tight manner, the hose barb component to the remaining part of the gas valve system and on the other side have a particular shape referred to as Christmas-tree shape, allowing to slide the tubing onto the hose barb component in an easy manner. This easy way of connecting tubing to the gas valve system, allows avoiding the need for a clamping ring for mounting tubing to the gas valve system and hence prevents loss of time when mounting the tubing to the gas valve system.

Depending on the flow rate applied and on local practice, in some cases a moisturizer, also referred to as humidifier, can be used between the gas valve system and the tubing providing the gas to the patient, to prevent drying up of the nasal mucous membrane. In those cases, the moisturizer typically is directly mounted on the gas valve using mutually matching threaded portions. In this case, the hose barb component, used for connecting the tubing of the nasal cannula or mouth mask to the remaining part of the gas valve system, typically is not used.

It has been found that hose barb components typically get lost, when switching between medical gas administration using a moisturizer and medical gas administration without using a moisturizer. This leads to loss of time for the medical staff and to the need of additional hose barb components in order to replace the lost hose barb components.

### Summary of the invention

It is an object of embodiments of the present invention to provide good gas valve systems and methods of using them.
The present invention relates to a gas valve system adapted for controlling the flow of a medical gas for a user, the gas valve system comprising an input portion for connecting the gas valve to an external supply, a regulating system for regulating the flow of the gas and an output portion for providing the controlled flow of the medical gas towards the user, the output portion having a tubular shaped ending with a threaded portion for releasably connecting a hose barb component suitable for connecting tubing to the gas valve, characterised in that the output portion furthermore has a protrusion upstream the threaded portion, and the system having a linking element being connected to the output portion of the gas valve system upstream the protrusion and being suitable for fixedly linking the hose barb component to the output portion of the gas valve system.
Embodiments of the present invention thus provide, when the hose barb component is mounted on the gas valve system a first releasable connection between the hose barb component and the gas valve via their threaded portions to provide gas flow from the gas valve through the hose barb component towards the user and a second fixed connection between the hose barb component and the gas valve system via the linking element for fixedly connecting the hose barb component to the gas valve, allowing for example to prevent loss of the hose barb component when the hose barb component is not in use, e.g. when a humidifier such as an aqua pack is connected to the gas valve.
It is an advantage of embodiments of the present invention that the hose barb is fixedly linked to the output portion so that, when the hose barb component is loosened from the threaded portion of the output portion, the hose barb component is still fixedly linked to the output portion and the hose barb component cannot be lost when it is not in use for connecting the gas valve system with tubing towards a user.
The protrusion may be ring-shaped and is extending around the output portion. It is an advantage of a protrusion that is extending around the output portion, e.g. along the circumference of the tubular shaped portion of the output portion, that the linking element cannot slip off the output portion and that the hose barb component thus has a good fixed connection to the output portion.
The linking element may comprise an opening with a diameter smaller than an outer diameter of the protrusion and the output portion may pass through the opening at the position upstream the protrusion of the output portion.
The linking element may be a flexible element. It is an advantage of embodiments of the present invention that the linking element is a flexible element allowing a more easy releasing the releasable connection of the hose barb component at its threaded portion.
The linking element may comprise a strip of solid flexible material. In some embodiments, the linking element may be made of a strip of solid flexible material.
The strip of solid flexible material may be made of any of a plastic material or a rubber material.
The linking element may comprise a chain.
The hose barb component may be a part of the gas valve system. The hose barb component may have a threaded portion for obtaining said releasable connection to the output portion via the threaded portion of the tubular shaped ending of the output portion and the hose barb component furthermore may be connected to the linking element for providing said fixed connection between the hose barb component and the output portion of the gas valve system.
10.- A gas valve system according to any of the previous claims, wherein the hose barb component comprises a portion with a Christmas tree shape.
Particular and preferred aspects of the invention are set out in the accompanying independent and dependent claims. Features from the dependent claims may be combined with features of the independent claims and with features of other dependent claims as appropriate and not merely as explicitly set out in the claims.
These and other aspects of the invention will be apparent from and elucidated with reference to the embodiment(s) described hereinafter.

### Brief description of the drawings

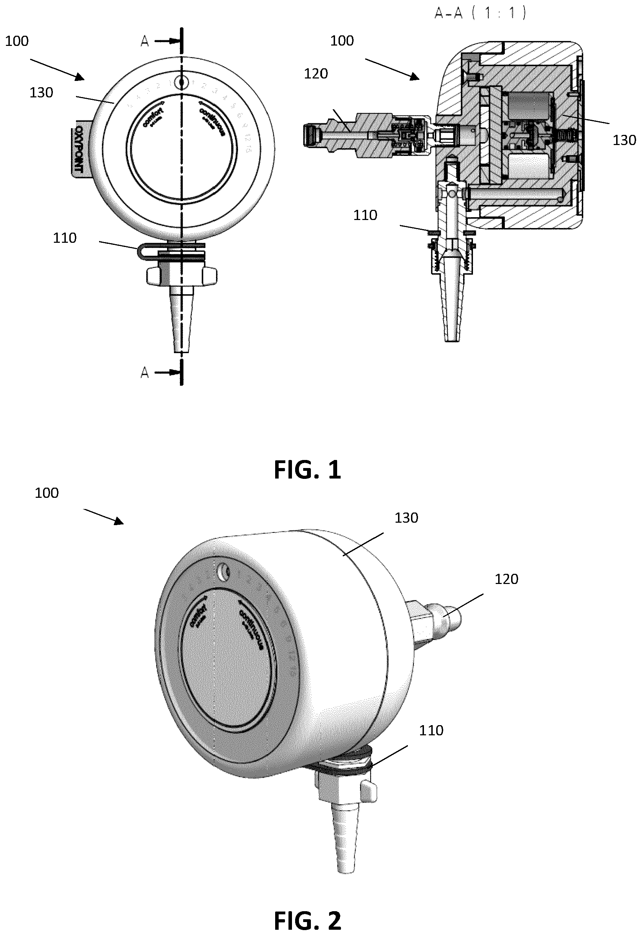

FIG. 1 to FIG. 5 illustrates gas valve systems according to embodiments of the present invention.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### Detailed description of illustrative embodiments

The present invention will be described with respect to particular embodiments and with reference to certain drawings but the invention is not limited thereto but only by the claims. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

In a first aspect, the present invention relates to a gas valve system adapted for controlling the flow of a medical gas for a user. The gas valve system may be for use in hospital, for use at home or may be for mixed use. The gas valve system may be for continuous flow and/or may be for on demand use. It may be a gas valve system as described for example in international patent application WO2012/153293, although embodiments are not limited thereto. The gas valve system may be based on only mechanical components or may be based on a combination of mechanical and electronical components. According to embodiments of the present invention, the gas valve system comprises an input portion for connecting the gas valve to an external supply. Such an external supply may for example be a pressurised gas bottle or may be a gas supply network as typically available in hospitals. The input portion may be as known from prior art and is therefore not further detailed here. The system also comprises a regulating system for regulating the flow of the medical gas. As indicated above, the present invention is not limited to the particular regulating system used. Such a system may for example be only mechanical or may comprise a combination of mechanical and electronic components. One possible example of a regulating system is described in WO2012/153293, although embodiments are not limited thereto. According to embodiments of the present invention, the gas valve system also comprises an output portion for providing the controlled flow of the medical gas towards the user. The medical gas may for example be administered to the user making use of a nasal cannula or a mouth mask. According to embodiments of the present invention, the output portion has a tubular shaped ending with a threaded portion for releasably connecting a hose barb component suitable for connecting tubing to the gas valve. The output portion furthermore has a protrusion upstream the threaded portion. The system further has a linking element being connected to the output portion of the gas valve system upstream the protrusion and being suitable for fixedly linking the hose barb component to the output portion of the gas valve system. The protrusion may be ring-shaped and extending around the output portion. The system thus typically may be configured such that the linking element is withheld from being removed from the remaining part of the gas valve system by the protrusion, e.g. withheld from being removed when no large force is applied to the linking element. A force is considered large if it is substantially higher than for example the gravitational force present when a hose barb component is connected to the linking element that is not attached to the gas valve system in another manner than through the linking element. Embodiments of the present invention provide, when the hose barb component is mounted on the gas valve system a first releasable connection between the hose barb component and the gas valve via their threaded portions to provide gas flow from the gas valve through the hose barb component towards the user and a second fixed connection between the hose barb component and the gas valve system via the linking element for fixedly connecting the hose barb component to the gas valve.

In some embodiments, the linking element may for example comprise an opening with a diameter smaller than an outer diameter of the protrusion and the output portion passes through the opening at the position upstream the protrusion of the output portion.

The linking element may be a flexible element. In some embodiments, the linking element comprises a strip of solid flexible material, for example strip of plastic material or a rubber material. In some embodiments, part of the linking element also may comprise a chain.

In some embodiments, the hose barb component is part of the gas valve system. The hose barb component typically may comprise a threaded portion for obtaining the releasable connection to the output portion via the threaded portion of the tubular shaped ending of the output portion. In these embodiments, the hose barb component also is connected to the linking element for providing said fixed link between the hose barb component and the output portion of the gas valve system. Such a connection may be performed in a variety of ways.

The hose barb component, whether it is part of the gas valve system or whether it is connectable to the gas valve system through the linking element, may comprise a portion with a Christmas tree shape, as well known by the person skilled in the art. More generally, the hose barb component may have at one side a shape suitable for easily connecting tubing such as tubing of a nasal cannula or a mouth mask.

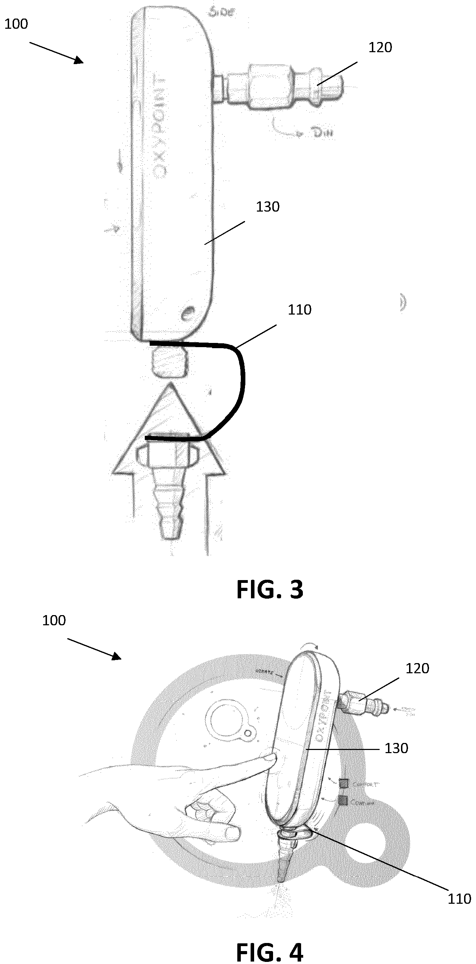

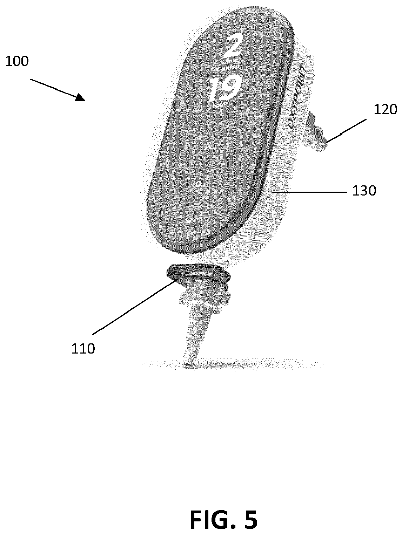

By way of illustration, embodiments of the present invention not being limited thereto, an example of an embodiment illustrating a gas valve system with a linking element is shown in FIG. 1. The gas valve system 100 is shown with linking element 110, connecting the hose barb component 120 fixedly to the remaining part of the gas valve system. The remaining gas valve system also shows the input portion 120 and the gas regulating system 130. The gas valve system is show in front view (left hand side) and in cross-sectional view (right hand side). The detailed features of the internal part of the remaining part of the gas valve system are not limiting for the present invention, as also indicated above. Furthermore, the explicit configuration of the linking element, such as the length, the way it is connected or connectable to the hose barb component, etc. is also not limiting. Similarly, also in FIG. 2 a gas valve system is shown, in the present case with a hose barb component connected to the remaining part of the gas valve. Furthermore, also in FIG. 3 a gas valve system with a linking element is shown. The hose barb component is not being releasably connected through the threaded portions but only is fixedly connected through the linking element 110. In this example, the input portion 120 and the gas regulating system 130 is also shown. FIG. 4 illustrates a similar gas valve system whereby the hose barb component is releasably connected to the remaining part of the gas valve system. FIG. 5 also shows a gas valve system according to an embodiment of the present invention.

In one aspect, the present invention also relates to a linking element adapted for use in a gas valve system adapted for controlling the flow of a medical gas for a user, a gas valve system as described in the first aspect.

## Claims

1. A gas valve system adapted for controlling the flow of a medical gas for a user, the gas valve system comprising
- an input portion for connecting the gas valve to an external supply,
- a regulating system for regulating the flow of the gas
- an output portion for providing the controlled flow of the medical gas towards the user, the output portion having a tubular shaped ending with a threaded portion for releasably connecting a hose barb component suitable for connecting tubing to the gas valve,
**characterised in that**
the output portion furthermore has a protrusion upstream the threaded portion, and the system having a linking element being connected to the output portion of the gas valve system upstream the protrusion and being suitable for fixedly linking the hose barb component to the output portion of the gas valve system.

2. A gas valve system according to claim 1, wherein the protrusion is ring-shaped and is extending around the output portion..

3. A gas valve system according to any of the previous claims, wherein the linking element comprises an opening with a diameter smaller than an outer diameter of the protrusion and the output portion passes through the opening at the position upstream the protrusion of the output portion.

4. A gas valve system according to any of the previous claims, wherein the linking element is a flexible element.

5. A gas valve system according to any of the previous claims, wherein the linking element comprises a strip of solid flexible material.

6. A gas valve system according to claim 5, wherein the strip of solid flexible material is made of any of a plastic material or a rubber material.

7. A gas valve system according to any of claims 1 to 4, wherein the linking element comprises a chain.

8. A gas valve system according to any of the previous claims, wherein the hose barb component is part of the gas valve system, the hose barb component having a threaded portion for obtaining said releasable connection to the output portion via the threaded portion of the tubular shaped ending of the output portion and the hose barb component furthermore being connected to the linking element for providing said fixed link between the hose barb component and the output portion of the gas valve system.

9. A gas valve system according to any of the previous claims, wherein the hose barb component comprises a portion with a Christmas tree shape.

10. A linking element adapted for use in a gas valve system adapted for controlling the flow of a medical gas for a user, the gas valve system according to any of claims 1 to 10.
